# EUROPEAN PATENT SPECIFICATION

(11) **EP 2 693 953 B1**
(45) Date of publication and mention of the grant of the patent: **04.07.2018**
(21) Application number: 12782539.6
(22) Date of filing: 05.04.2012
(51) Int. Cl.: A61B 8/06, G01B 9/02, A61B 6/03

(54) **MULTI-MODALITY MEDICAL SENSING SYSTEM AND METHOD**
MULTIMODALES MEDIZINISCHES ERFASSUNGSSYSTEM UND -VERFAHREN
SYSTÈME ET MÉTHODE DE DÉTECTION MÉDICALE À MULTIMODALITÉ

(30) Priority: 08.04.2011 US 201161473570 P
(43) Date of publication of application: 12.02.2014
(73) Proprietor: Volcano Corporation, San Diego, CA 92130 (US)
(72) Inventor: DE JONG, Duane, Elk Grove, CA 95624 (US); LITZZA, Gerald L., Sacramento, CA 95864 (US); SCHUERKAMP, Dennis, North Highlands, CA 95660 (US); HOSEIT, Paul Mitchell, El Dorado Hills, CA 95762 (US)
(74) Representative: de Haan, Poul Erik
(86) International application number: PCT/US2012/032335
(87) International publication number: WO 2012/154335

(56) References cited:
- JP-A- 2002 541 563
- JP-A- 2008 293 189
- US-A1- 2003 216 621
- US-A1- 2006 173 246
- US-A1- 2006 173 246
- US-A1- 2008 269 572
- US-A1- 2011 087 664

## Description

### TECHNICAL FIELD

Embodiments of the present disclosure relate generally to the field of medical devices and, more particularly, to a multi-modality medical sensing system and associated methods of use.

### BACKGROUND

Innovations in diagnosing and verifying the level of success of treatment of disease have migrated from external imaging processes to internal diagnostic processes. In particular, diagnostic equipment and processes have been developed for diagnosing vasculature blockages and other vasculature disease by means of ultra-miniature sensors placed upon the distal end of a flexible elongate member such as a catheter, or a guide wire used for catheterization procedures. For example, known medical sensing techniques include angiography, intravascular ultrasound (IVUS), forward looking IVUS (FL-IVUS), fractional flow reserve (FFR) determination, a coronary flow reserve (CFR) determination, optical coherence tomography (OCT), trans-esophageal echocardiography, and image-guided therapy. Each of these techniques may be better suited for different diagnostic situations. To increase the chance of successful treatment, health care facilities may have a multitude of imaging and sensing modalities on hand in a catheter lab during a procedure. However, each imaging modality in a catheter lab traditionally requires its own special-purpose diagnostic equipment. For instance, an imaging modality may require a catheter, a patient isolation module (PIM), a user control interface, a display, a specialized power unit, and a processing unit such as a customized personal computer. Traditionally, all of this equipment is located in the catheter room itself during a procedure and depends on a substantial wiring infrastructure for connectivity and dependable power. Physical space is typically at a premium in catheter labs and each additional imaging modality employed in a catheter lab complicates the pre -procedure setup and limits the movement of health care professionals during procedures. Additionally, each imaging modality device must be independently setup and managed by an clinician trained to operate the unique controls of the specific devices. This may not be convenient, given the limits of patient safety, procedure time, staffing and the availability of sufficiently trained personnel. Further, current integrated software
solutions that combine multiple imaging modalities are difficult to upgrade and are otherwise problematic.

US 2006/0173246 A1 describes a mobile point-of-care medical station adapted to be employed by patient monitors made by a variety of different manufacturers. A mobile point-of-care medical station includes a patient medical parameter processor and a communication interface. The patient medical parameter processor automatically acquires data, representing medical parameters of a patient, and processes the patient medical parameter data for presentation to a user on a display. The communication interface enables communication with remote systems via a network and communicates patient medical parameter data to a remote system by formatting patient medical parameter data into multiple individual messages. An individual message incorporates a patient identifier and a communication address. The communication address is associated with a particular communication interface of a particular mobile point-of-care medical station, and enables the remote system to identify the particular mobile point-of-care medical station from multiple different mobile point-of-care medical stations.

In US 2003/0216621 A1, a multifunctional invasive cardiovascular diagnostic measurement host is disclosed that interfaces a variety of sensor devices, such as guide wire-mounted pressure sensors, flow sensors, temperature sensors, etc, and provides a multi-mode graphical user interface providing a plurality of displays in accordance with the various types of sensors and measurements rendered by the sensors.

US 2008/0269572 A1 provides a multipurpose host system for processing and displaying invasive cardiovascular diagnostic measurement data. The system includes a an external input signal bus interface. The bus interface receives data arising from cardiovascular diagnostic measurement sensors. Measurement processing components receive data from particular sensor types. Based on the received data, the processing components render diagnostic measurement parameter values. A multi-mode graphical user interface includes display components corresponding to data received from particular sensor types. The user interface provides recommended action prompts that guide a user through a series of actions.

While the existing devices and methods have been generally adequate for their intended purposes, they have not been entirely satisfactory in all respects. The medical sensing systems and associated methods of the present disclosure overcome one or more of the shortcomings of the prior art.

### SUMMARY

The problems mentioned above are solved by a medical sensing system as defined in claim 1. Particular embodiments of the invention are defined in dependent claims 2 to 13.

### BRIEF DESCRIPTION OF THE DRAWINGS

Figure 1 is a schematic drawing depicting a medical sensing system including a multi-modality processing system according to one embodiment of the present disclosure.
Figure 2 is a functional block diagram of a software framework executing on an embodiment of the multi-modality processing system in the medical sensing system.
Figure 3 is an illustration of a message format utilized by the multi-modality processing system in one embodiment of the present disclosure.
Figure 4 is a high-level flowchart illustrating a method of processing medical sensing data in the multi-modality processing system.
Figure 5 is a schematic drawing depicting a medical sensing system including a multi-modality processing system according to another embodiment of the present disclosure.
Figure 6 is a diagrammatic perspective view of an aspect of the medical sensing system of Figure 5, namely, the multi-modality processing system.

### DETAILED DESCRIPTION

For the purposes of promoting an understanding of the principles of the present disclosure, reference will now be made to the embodiments illustrated in the drawings, and specific language will be used to describe the same. It will nevertheless be understood that no limitation of the scope of the disclosure is intended. Any alterations and further modifications in the described devices, instruments, methods, and any further application of the principles of the disclosure as described herein are contemplated as would normally occur to one skilled in the art to which the disclosure relates. In particular, it is fully contemplated that the features, components, and/or steps described with respect to one embodiment may be combined with the features, components, and/or steps described with respect to other embodiments of the present disclosure.

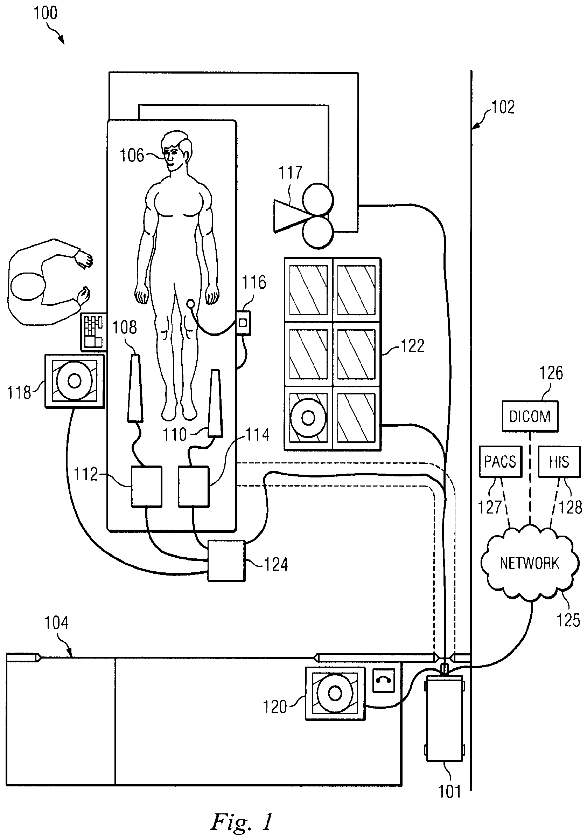

Figure 1 is a schematic drawing depicting a medical sensing system 100 including a multi-modality processing system 101 according to one embodiment of the present disclosure. In general, the medical sensing system 100 provides for coherent integration and consolidation of multiple forms of acquisition and processing elements designed to be sensitive to a variety of methods used to acquire and interpret human biological physiology and morphological information. More specifically, in system 100, the multi-modality processing system 101 is an integrated device for the acquisition, control, interpretation, and display of multi-modality medical sensing data. In one embodiment, the processing system 101 is a computer workstation with the hardware and software to acquire, process, and display multi-modality medical sensing data, but in other embodiments, the processing system 101 may be any other type of computing system operable to process medical sensing data. In the embodiments in which processing system 101 is a computer workstation, the system includes at least a processor such as a microcontroller or a dedicated central processing unit (CPU), a non-transitory computer-readable storage medium such as a hard drive, random access memory (RAM), and/or compact disk read only memory (CD-ROM), a video controller such as a graphics processing unit (GPU), and a network communication device such as an Ethernet controller.

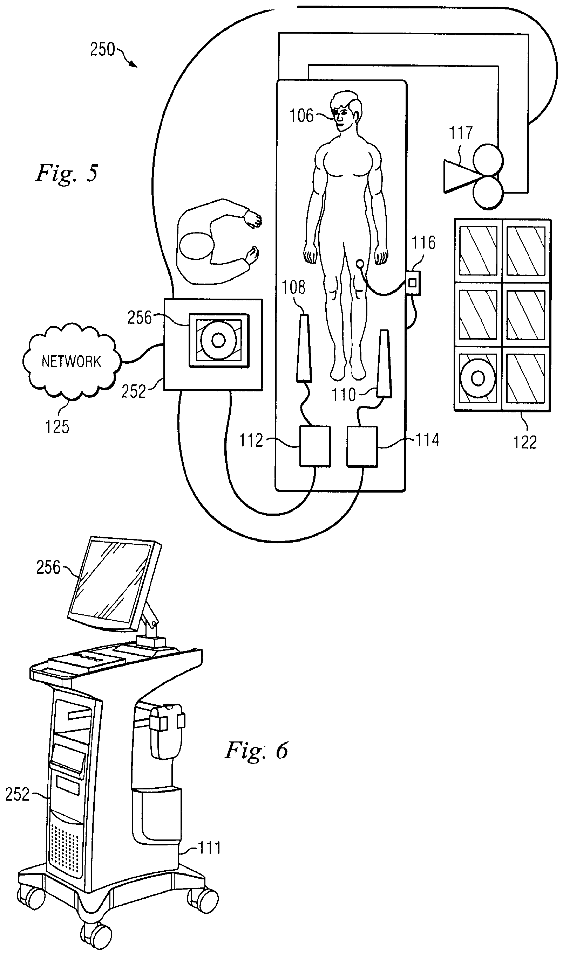

In the illustrated embodiment, the medical sensing system 100 is deployed in a catheter lab 102 having a control room 104, with the processing system 101 being located in the control room. In other embodiments, the processing system 101 may be located elsewhere, such as in the catheter lab 102 itself as will be described in association with Figures 5 and 6. The catheter lab 102 includes a sterile field but its associated control room 104 may or may not be sterile depending on the requirements of a procedure and/or health care facility. The catheter lab and control room may be used to perform on a patient any number of medical sensing procedures such as angiography, intravascular ultrasound (IVUS), virtual histology (VH), forward looking IVUS (FL-IVUS), intravascular photoacoustic (IVPA) imaging, a fractional flow reserve (FFR) determination, a coronary flow reserve (CFR) determination, optical coherence tomography (OCT), computed tomography, intracardiac echocardiography (ICE), forward-looking ICE (FLICE), intravascular palpography, transesophageal ultrasound, or any other medical sensing modalities known in the art. For example, in catheter lab 102 a patient 106 may be undergoing a multi-modality procedure either as a single procedure or in combination with one or more sensing procedures, in which IVUS data will be collected with an IVUS catheter 108 and OCT data will be collected with an OCT catheter 110. The IVUS catheter 108 may include one or more sensors such as a phased-array transducer. In some embodiments, the IVUS catheter 108 may be capable of multi-modality sensing such as IVUS and IVPA sensing. The OCT catheter 110 may include one or more optical sensors. In the embodiment illustrated in Figure 1, the medical sensing system 100 includes a number of interconnected medical sensing-related tools in the catheter lab 102 and control room 104 to facilitate this multi-modality workflow procedure, including an IVUS patient isolation module (PIM) 112, an OCT PIM 114, an electrocardiogram (ECG) device 116, an angiogram system 117, a bedside control surface 118, a control room control surface 120, and a boom display 122. A bedside utility box (BUB) 124 in the catheter lab 102 acts as a hub for the PIMs 112 and 114, ECG device 116, and bedside control surface 118 and communicatively couples them to the processing system 101. In the illustrated embodiment, the angiography system 117, control room control surface 120, and boom display 122 are communicatively coupled directly to the processing system 101. However, in alternative embodiments, these tools may be coupled to the processing system 101 via the BUB 124. In one embodiment, the BUB 124 is a passive cable pass-through device that consolidates wires and feeds them into an underfloor cabling trench, but, alternatively, in other embodiments, the BUB 124 may contain logic and communication circuitry to actively coordinate communication between the medical sensing tools and the processing system 101. U.S. Provisional Patent Application No. 61/473,625, entitled "MEDICAL SENSING COMMUNICATION SYSTEM AND METHOD" and filed on April 8, 2011, discloses a bedside utility box that intelligently couples medical sensing-related tools. Further, the multi-modality processing system 101 is communicatively coupled to a data network 125. In the illustrated embodiment, the data network 125 is a TCP/IP-based local area network (LAN), however in other embodiments, it may utilize a different protocol such
as Synchronous Optical Networking (SONET), or may be a wide area network (WAN). The processing system 101 may connect to various resources via the network 125. For example, the processing system 101 may communicate with a Digital Imaging and Communications in Medicine (DICOM) system 126, a Picture Archiving and Communication System (PACS) 127, and a Hospital Information System 128 through the network 125.

Additionally, in the illustrated embodiment, medical sensing tools in system 100 are communicatively coupled to the processing system 101 via a wired connection such as a standard copper link or a fiber optic link, but, in alternative embodiments, the tools may be connected to the processing system 101 via wireless connections using IEEE 802.11 Wi-Fi standards, Ultra Wide-Band (UWB) standards, wireless FireWire, wireless USB, or another high-speed wireless networking standard.

In the medical sensing system 100, the IVUS PIM 112 and OCT PIM 114 are operable to respectively receive medical sensing data collected from the patient 106 by the IVUS catheter 108 and OCT catheter 110 and are operable to transmit the received data to the processing system 101 in the control room 104. In one embodiment, the IVUS PIM 112 and OCT PIM 114 transmit the medical sensing data over a Peripheral Component Interconnect Express (PCIe) data bus connection, but, in other embodiments, they may transmit data over a USB connection, a Thunderbolt connection, a FireWire connection, or some other high-speed data bus connection. In one embodiment, the PIMs 112 and 114 include analog to digital (A/D) converters and transmit digital data to the processing system 101, however, in other embodiments, the PIMs transmit analog data to the processing system. Additionally, the ECG device 116 is operable to transmit electrocardiogram signals or other hemodynamic data from patient 106 to the processing system 101. In some embodiments, the processing system 101 may be operable to synchronize data collection with the catheters 108 and 110 using ECG signals from the ECG 116. Further, the angiogram system 117 is operable to collect x-ray, computed tomography (CT), or magnetic resonance images (MRI) of the patient 106 and transmit them to the processing system 101. In one embodiment, the angiogram system 117 may be communicatively coupled to the processing system 101 through the network 125, but, in other embodiments, the angiogram system may be more directly coupled to the processing system 101, for example through an adapter device. Such an adaptor device may transform data from a proprietary third-party format into a format usable by the processing system 101. In some embodiments, the processing system 101 may co-register image data from angiogram system 117 (e.g. x-ray data, MRI data, CT data, etc.) with sensing data from the IVUS and OCT catheters 108 and 110. As one aspect of this, the co-registration may be performed to generate three-dimensional images with the sensing data.

The bedside control surface 118 is also communicatively coupled to the processing system 101 via the BUB 124 and provides user control of the particular medical sensing modality (or modalities) being used to diagnose the patient 106. In the current embodiment, the bedside control surface 118 is a touch screen that provides user controls and diagnostic images on a single surface. In alternative embodiments, however, the bedside control surface 118 may include both a non-interactive display and separate controls such as physical buttons and/or a joystick. In the integrated medical sensing system 100, the bedside control surface 118 is operable to present workflow control options and patient image data in graphical user interfaces (GUIs). The bedside control surface 118 is capable of displaying GUIs for multiple modalities, and thus a clinician does not have to physically move between user interface devices when switching sensing modalities.

The control room control surface 120 in the control room 104 is also communicatively coupled to the processing system 101 and, as shown in Figure 1, is adjacent to catheter lab 102. In the current embodiment, the control room control surface 120 is similar to the bedside control surface 118 in that it includes a touch screen and is operable to display multitude of GUI-based workflows corresponding to different medical sensing modalities. In some embodiments, the control room control surface 120 may be used to simultaneously carry out a different aspect of a procedure's workflow than the bedside control surface 118. In alternative embodiments, the control room control surface 120 may include a non-interactive display and standalone controls such as a mouse and keyboard.

The system 100 further includes the boom display 122 communicatively coupled to the processing system 101. The boom display 122 may include an array of monitors, each capable of displaying different information associated with a medical sensing procedure. For example, during an IVUS procedure, one monitor in the boom display 122 may display a tomographic view and one monitor may display a sagittal view.

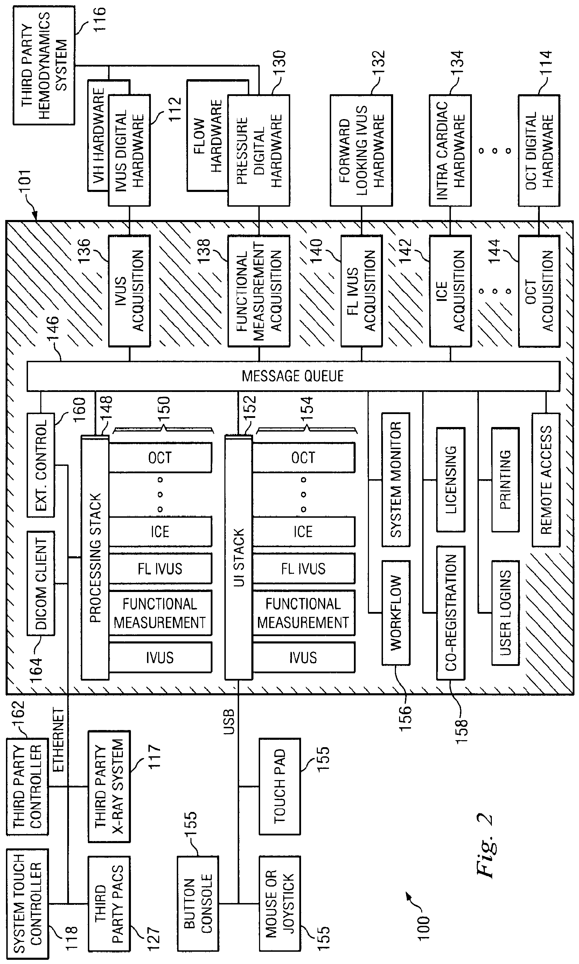

With reference now to Figure 2, illustrated is a functional block diagram of the software framework executing on an embodiment of the multi-modality processing system 101 in the medical sensing system 100. In general, the software framework of processing system 101 is modular and extensible. That is, the software framework is driven by independent software modules respectively associated with a specific medical sensing modality. This modular design allows the framework to be extended to accommodate additional medical sending modalities without an impact on existing functionality. Further, an internal messaging system facilitates independent data communication between software modules in the framework.

In more detail, in the embodiment shown in Figure 2, the processing system 101 is communicatively coupled and operable to receive medical sensing data from a plurality of medical sensing devices. For example, the IVUS PIM 112, the OCT PIM 114, the ECG system 116, and a fractional flow reserve (FFR) PIM 130 are communicatively coupled to the system 101 via a PCIe connection. Additionally, a FLIVUS PIM 132 is communicatively coupled to the system 101 via a USB connection and a ICE PIM 134 is communicatively coupled to the system 101 via a FireWire connection. In other embodiments, additional and/or different medical sensing devices may be coupled to the processing system 101 via additional and/or different data communication connections known in the art. As mentioned above, some PIMs may send analog medical sensing data to the processing system 101. In such a case, medical sensing data may be digitized as it arrives, for example, by the PCIe hardware onboard the system 101.

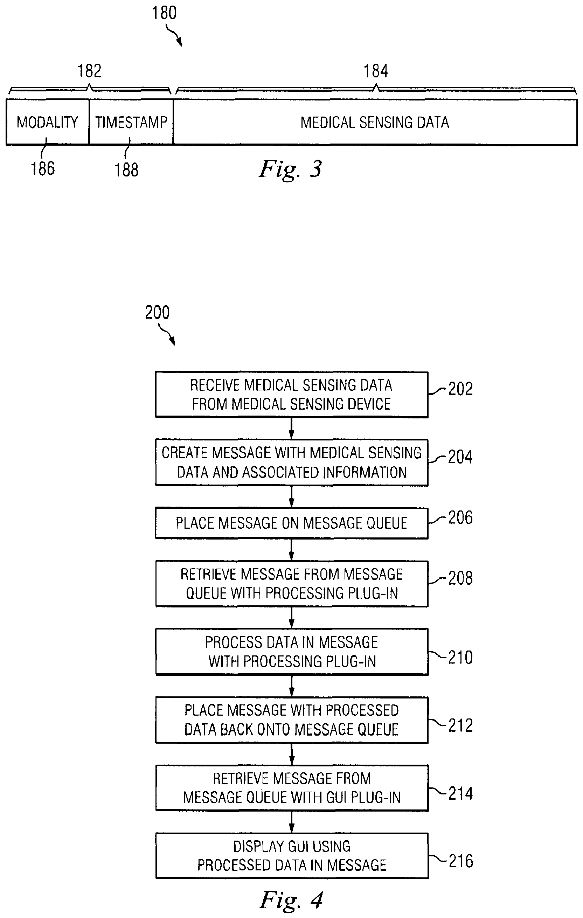

To facilitate communication between the processing system 101 and the medical sensing devices coupled thereto, the software framework includes a plurality of data acquisition modules, each associated with a respective modality (i.e. medical sensing device). For example, an IVUS acquisition module 136 is operable to receive IVUS data from the IVUS PIM 112 via the PCIe bus. Likewise, a FFR acquisition module 138 is operable to receive FFR data from the FFR PIM 130 via the PCIe bus, a FLIVUS acquisition module 140 is operable to receive FLIVUS data from the FLIVUS PIM 132 via the USB bus, an ICE acquisition module 142 is operable to receive ICE data from the ICE PIM 134 via the FireWire bus, and an OCT acquisition module 144 is operable to receive OCT data from the OCT PIM 114 via the PCIe bus. Once the acquisition modules 136, 138, 140, 142, and 144 have received data from the connected medical sensing devices, the modules packetize the data to facilitate intersystem communication. Specifically, the modules are operable to create a plurality of messages from the incoming digital data stream, where each message contains a portion of the digitized medical sensing data and a header. The message header contains information associated with the medical sensing data contained within the message. The format of these messages will be described in more detail in association with Figure 3. Further, in some embodiments, the acquisition modules 136, 138, 140, 142, and 144 may be operable to manipulate the digitized medical sensing data in some way before it is packetized. For example, the acquisition modules may compress the sensing data to make intersystem communication more efficient, or normalize, scale or otherwise filter the data to aid later processing of the data. In some embodiments, this manipulation may be modality-specific. For example, the OCT acquisition module 144 may identify and discard redundant OCT data before it is packetized to save processing time down the line. The acquisition modules 136, 138, 140, 142, and 144 may additionally perform a number of tasks related to the acquisition of data including responding to interrupts generated by the data buses (e.g. PCIe, USB), detecting which medical sensing devices are connected to processing system 101, retrieving information about connected medical sensing devices, storing sensing device-specific data, and allocating resources to the data buses. In accordance with the modular architecture of the software framework, the data acquisition modules are independent from each other and may be installed or removed without disrupting data acquisition by other modules. Additionally, acquisition modules are independent of the underlying data bus software layers (e.g. through the use of Application Programming Interfaces (APIs)) and thus may be written by third parties to acquire data from third party medical sensing devices.

The software framework further includes a message queue 146 to facilitate intersystem communication between different components in the processing system 101. The message queue 146 is operable to receive messages from software modules within the framework of system 101, temporarily store the messages, and make the messages available for retrieval by software modules within the framework. For instance, after the acquisition modules 136, 138, 140, 142, and 144 packetize incoming medical sensing data into messages, they pass the messages to the message queue 146, where they wait for further processing. In one embodiment, the message queue 146 includes modality-specific queues for messages associated with each sensing modality. For example, in such a scenario, a portion of the message queue 146 may be allocated for messages containing IVUS data received from the IVUS data acquisition module 136. Further, in some embodiments, the message queue 146 is operable to make received messages available in a First-In-First-Out (FIFO) manner, wherein messages that arrive on the bus first will be removed from the bus first. In alternative embodiments, the message queue 146 may make messages available in a different manner for instance by a priority value stored in the message header. In one embodiment, the message queue 146 is implemented in random-access memory (RAM) in the processing system 101, but, in other embodiments, the message queue may be implemented in non-volatile RAM (NVRAM), secondary storage (e.g. magnetic hard drives, flash memory, etc), or network-based storage. Further, in one embodiment, messages stored on the message queue 146 may be accessed by software and hardware modules in processing system 101 using Direct Memory Access (DMA).

The software framework of processing system 101 further includes a processing software stack 148 that supports a layer of processing plug-ins (or modules) 150. In general, each processing plug-in in the layer 150 corresponds to a medical sensing modality and processes data associated with that particular sensing modality. For example, an IVUS processing plug-in in layer 150 may interpret IVUS data received from the IVUS PIM 112 and convert the data into displayable IVUS images. In one embodiment, the software stack 148 may expose a set of application programming interfaces (APIs) with which the plug-ins in the layer 150 may call to access system resources such as the message queue 146, computational resources, and communication resources. Further, in some embodiments, the plug-in layer 150 may include co-registration plug-ins, in which medical sensing data from a plurality of medical sensing devices are interpreted and co-registered. To support such co-registration applications, in one embodiment, the software stack 148 may expose time synchronization APIs for use by the plug-ins and may expose APIs through which the processing modules may access specialized co-registration modules, databases containing co-registration data, or sensing systems producing real-time data for use in co-registration, such as angiography system 117 or an MRI system.

The processing plug-ins in the layer 150 are operable to retrieve messages on the message queue 146 that contain unprocessed medical sensing data. For example, using one or more APIs, a plug-in associated with a sensing modality may query the message queue 146 to determine if messages corresponding to the sensing modality are waiting to be processed on the bus. If so, the plug-in may retrieve one or more of the oldest messages (in those embodiments using FIFO) and process the raw data therein. After processing the data, the plug-in may re-packetize the data and place a message containing the processed data back on the message queue 146. In some embodiments, before sending the message to the bus, the plug-ins may insert a flag in the header indicating that the message contains processed data. In other embodiments, however, rather than insert a flag, the plug-in may instead place the messages in a queue in the message queue 146 that is allocated for messages containing processed data, or perform some other operation to indicate that the medical sensing data in the messages has been processed. Additionally, in some embodiments, after processing medical sensing data, the plug-ins may transmit the processed data to archival systems such as a locally attached mass storage device or the network-based PACS server 127.

In accordance with the modular architecture of the software framework, the processing plug-ins in the layer 150 are independent of each other and the software stack 148 and may be installed or removed without disrupting other plug-ins, and may be written by third parties. Further, they are operable to process signaling and imaging data from multiple medical sensing devices concurrently. In this regard, in some embodiments, the processing software stack 148 may intelligently make use of the computing resources by identifying generic computing tasks common to concurrently executing processing plug-ins. For example, the processing software stack 148 may determine that two processing plug-ins (associated with different modalities) each need to perform filtering, image processing, and scan conversion processes on incoming sensing data. Once these common tasks are identified, the software stack 148 may utilize a library of parallel algorithms to process these tasks concurrently. The software framework of processing system 101 further includes a user interface software stack 152 that supports a layer of graphical user interface (GUI) plug-ins (or modules) 154. In general, each GUI plug-in in the layer 154 corresponds to a medical sensing modality and is operable to render a user interface for control of the associated acquisition workflow and display of processed sensing data. In one embodiment, the user interface software stack 152 may expose APIs with which the plug-ins in the plug-in layer 154 may call to access system resources such as the message queue 146, a look-and-feel toolbox, and error handling resources. The look-and-feel toolbox APIs enable the GUI plug-ins to present a standardized user interface with common buttons, parallel workflow formats, and data presentation schemes. In this manner, clinicians may more easily transition between acquisition modalities without additional user interface training. Further, co-registration GUI plug-ins in layer 154 may present and/or combine processed image or signaling data from multiple modalities. For instance, a GUI plug-in may display an electrocardiogram (ECG) wave adjacent to IVUS imaging data or may display an IVUS image overlaid with borders that were previously drawn on an OCT image. Further, in some embodiments, the UI software stack 152 may include a multi-tasking framework to coordinate concurrently executing GUI plug-ins. For instance, in the event the processing system 101 is simultaneously acquiring data associated with more than one modality, the UI software stack may present the user with a modality selector screen on which a desired GUI may be selected.

The GUI plug-ins in layer 154 are operable to retrieve messages from the message queue 146 that contain processed medical sensing data. For example, using one or more APIs, a plug-in associated with a sensing modality may query the message queue 146 to determine if messages associated with the sensing modality are waiting on the bus. If so, the plug-in may retrieve one or more of the oldest messages (in those embodiments using FIFO) and render the processed data therein using the look-and-feel toolbox APIs. The rendered GUI may then be displayed on one or more control/display device such as bedside control surface 118, control room control surface 120, and boom display 122. Further, in some embodiments, additional control devices 155 (e.g. button consoles, mice, keyboards, touch pads) may be communicatively coupled to the processing system 101 to facilitate user interaction with the GUIs. In some embodiments, the processing system 101 may make the rendered GUIs accessible by remote network-connected devices via data network 125. In this manner, a clinician outside of the catheter lab 102 may control a data acquisition workflow or passively monitor medical sensing images. Further, in some embodiments, the processing system 101 may be operable to output independent GUIs to different control devices such that clinicians may perform multiple workflows using the same sensing data simultaneously. Additionally, in accordance with the modular architecture of the software framework, the GUI plug-ins in the layer 154 are independent of each other and the software stack 152 and may be installed or removed without disrupting other plug-ins, and may be written by third parties.

The software framework further includes additional software modules to facilitate multi-modality data acquisition and processing. For instance, the processing system includes a workflow module 156. In general, the workflow module 156 is operable to set up and coordinate data acquisition workflows for processing system 101. In some embodiments, the workflow module 156 may be operable to communicate with the DICOM server 126 or HIS 128 via the network 125 to retrieve patient procedure information, such as scheduling information. Also, the workflow module 156 may report the sensing capabilities of the processing system 101 to the DICOM server 126 or HIS 128 so that appropriate workflows are assigned to processing system 101. Further, in some embodiments, the workflow module 156 may be operable to associate patient information and procedure with messages stored on the message queue 146. For instance, the module 156 may retrieve patient information from the DICOM server 126 and insert the information into the header of messages containing data acquired from the patient before the messages are archived.

Further, the software framework on processing system 101 includes a co-registration module 158. In general, the co-registration module 158 facilitates co-registration of medical sensing data associated with different sensing modalities. For instance, the module 158 may coordinate the acquisition of data from the angiography system 117 with the acquisition of data from the IVUS PIM 112. In some embodiments, the angiography system 117 may transmit messages containing x-ray imaging data to the message queue 146, and the co-registration module 158 may retrieve these messages, extract the data, and co-register it with data extracted from messages placed on the message queue by one of the data acquisition modules 136, 138, 140, 142, and 144. Still further, in other embodiments, the co-registration module 158 may be operable to spatially co-register catheter-gathered data in a two-dimensional or three-dimensional space using previously-generated 2D images or 3D models. For example, a catheter-based sensing tool may include fiducials that are tracked to generate position data during a sensing procedure, and the co-registration module 158 may register this position data against previously acquired MRI data.

Additionally, in other embodiments, the co-registration module 158 may be operable to facilitate time synchronization among medical sensing devices connected to processing system 101 for co-registration purposes. For instance, in one embodiment, the co-registration module 158 may be operable to serve as a master time server for the PIMs 112, 130, 132, 134, and 114 using a network-based time synchronization protocol such as the Precision Time Protocol (PTP) or the Network Time Protocol (NTP). In another embodiment, the co-registration module 158 may be operable to assign a common timestamp to data as it arrives on the message queue 146 from a plurality of medical sensing devices. Still further, in other embodiments, the processing system 101 may include a dedicated real-time clock to synchronize sampling by connected medical sensing devices. In such an embodiment, the co-registration module 158 may utilize the real-time clock to distribute a synchronization signal to connected sensing devices. In some embodiments, the real-time clock may be integrated into the co-registration module 158. U.S. Provisional Patent Application No. 61/473,591, entitled "DISTRIBUTED MEDICAL SENSING SYSTEM AND METHOD" and filed on April 8, 2011, discloses temporally synchronizing medical sensing data collection in more detail and is hereby incorporated by reference in its entirety.

The software framework on processing system 101 additionally includes an external controller module 160 to facilitate communication with a third-party controller 162. In one embodiment, the controller module 160 is operable to export control functionality of the processing system 101 to the third party controller 162, so that the controller 162 may present control functions in its user interface. Further, a DICOM client module 164 in the processing system 101 may facilitate communication with the DICOM server 126.

With reference now to Figure 3, illustrated is a message format utilized by the multi-modality processing system 101 in one embodiment of the present disclosure. As mentioned above, the data acquisition modules 136, 138, 140, 142, and 144 may convert incoming medical sensing data into a plurality of messages containing digitized sensing data and associated identifying information. Figure 3 illustrates an example message 180. The message 180 includes a header 182 and a payload 184. The payload 184 includes digitized medical sensing data collected with the medical sensing devices coupled to the processing system 101. The header 182 includes information associated with the sensing data in the payload 184. In the illustrated embodiment, the header 182 includes blocks 186 and 188 that respectively contain modality information associated with the data, and a timestamp. The modality information in block 186 may include the modality of the data in payload 184 (e.g. IVUS, OCT, etc.) and, in some embodiments, may also include information about the specific medical sensing device (e.g. catheter, PIM, etc.) with which the data was taken. Further, the timestamp in block 188 may correspond to the point in time the data in payload 184 was collected. In some embodiments, the timestamp value may be provided by the originating PIM, but in other embodiments, the timestamp may be provided by the co-registration module 158, or the data acquisition module responsible for creating the messages. The timestamp (or sequence number) in block 188 may be used to facilitate temporal co-registration of the sensing data in payload 184 with data of a different modality. In other embodiments, the header 182 may include additional information associated with the data in payload 184 such as procedure date and time, procedure location (e.g. health care facility, catheter lab, etc.), patient identification (e.g. name, social security number, date of birth, etc.), and doctor identification (e.g. name, license number, etc.), analog to digital conversion information, compression information, a flag indicating the processing status of the data, and workflow identification information.

Figure 4 is a high-level flowchart illustrating a method 200 of processing medical sensing data in multi-modality processing system 101. More specifically, method 200 describes the manner in which data messages flow within modular architecture of the processing system 101. For the sake of clarity, the following example will focus on the acquisition of IVUS data, but may similarly apply to other sensing modalities supported by processing system 101. Method 200 begins at block 202 where a stream of unprocessed IVUS data from the IVUS PIM 112 is received by the IVUS data acquisition module 136. In this example, IVUS data was digitized by the IVUS PIM 112 and thus arrives in digital form. Method 200 proceeds to block 204 where the IVUS data acquisition module 136 packetizes the data stream. Specifically, the data acquisition module 136 creates a plurality of messages that contain a portion of the data, information identifying the modality of the data, and a timestamp. Next, method 200 continues to block 206, where the IVUS data acquisition module 136 places the IVUS messages on the message queue 146. Then, in block 208, the IVUS processing plug-in in the software layer 150 retrieves the IVUS messages from the message queue 150 in the order in which they were placed on the bus (i.e. FIFO). Method 200 then proceeds to block 210 where the IVUS processing plug-in extracts the IVUS data from the messages, interprets the data, and renders IVUS images using the APIs exposed by the processing software stack 148. Next, in block 212, the IVUS processing plug-in re-packetizes the processed IVUS data and places it back on the message queue 146. In some embodiments, the IVUS plug-in also transmits the processed IVUS images to be archived in a storage device such as PACS server 127. Method 200 then proceeds to block 214 where the IVUS GUI plug-in in the software layer 154 retrieves the messages containing processed IVUS data from the message queue 146 in the order in which they were placed into the bus by the IVUS processing plug-in. Finally, method 200 proceeds to block 216 where the IVUS GUI plug-in extracts the IVUS image data and displays it as part of the IVUS workflow GUI on the bedside control surface 118 or other controller. Again, the IVUS message flow described above is just an example, but it may be representative of the message flow of data associated with other sensing modalities in processing system 101.

With reference now to Figures 5 and 6, Figure 5 is a schematic drawing depicting a medical sensing system 250 including a multi-modality processing system 252 according to another embodiment of the present disclosure. Figure 6 is a diagrammatic perspective view of the multi-modality processing system 252 of Figure 5.

Like the processing system 101 in system 100, the multi-modality processing system 252 in medical sensing system 250 is an integrated device for the acquisition, control, interpretation, and display of multi-modality medical sensing data. In this regard, the processing system 250 contains a similar software framework as processing system 101 (as shown in Figure 2). However, the processing system 252 is mobile and may be moved between catheter labs. In the illustrated embodiment, the processing system 250 is currently located in catheter lab 102 to perform an IVUS and OCT workflow on patient 106. In the medical sensing system 250, the IVUS PIM 112, OCT PIM 114, ECG system 116, angiography system 117, boom display 122, and data network 125 are communicatively coupled to the processing system 250. Although the medical sensing tools in system 250 are shown as communicatively coupled to each other and the processing system 252 via a wired connection (e.g. standard copper link, a fiber optic link), the tools may be connected to the processing system 252 via wireless connections (e.g. IEEE 802.11 Wi-Fi, UWB, wireless FireWire, wireless USB) in other embodiments.

Further, as shown in Figure 6, the processing system 252 sits on a wheeled cart 254 to facilitate the mobility of the system. In some embodiments, the cart 254 may include wire management systems to control the various wires attached to the system 252. Additionally, a controller 256 sits on the cart 254 and is communicatively coupled to the processing system 252. The controller 256 is operable to present a GUI to a clinician conducting a workflow. In the illustrated embodiment, the controller 256 is a touch screen that provides user controls and diagnostic images on a single surface. In alternative embodiments, however, the controller 256 may include both a non-interactive display and separate controls such as physical buttons and/or a joystick.

Although illustrative embodiments have been shown and described, a wide range of modification, change, and substitution is contemplated in the foregoing disclosure and in some instances, some features of the present disclosure may be employed without a corresponding use of the other features. For example, in some embodiments, the multi-modality processing systems 101 and 252 may be used to process non-cardiovascular diagnostic data such as data from cranial or peripheral arteries, as well as data from non-vascular body portions. Further, the systems 101 and 252 may be used to collect and process MRI data, or may be utilized in computer assisted surgery (CAS) applications. Further, the modules described above in association with the multi-modality processing systems may be implemented in hardware, software, or a combination of both. And the processing systems may be designed to work on any specific architecture. For example, the systems may be executed on a single computer, local area networks, client-server networks, wide area networks, internets, hand-held and other portable and wireless devices and networks. It is understood that such variations may be made in the foregoing without departing from the scope of the present disclosure. Accordingly, it is appropriate that the appended claims be construed broadly and in a manner consistent with the scope of the present disclosure.

## Claims

1. A medical sensing system (100) comprising a processing system (101) communicatively coupled to a plurality of medical sensing devices (108, 110, 116), the processing system including:
a data acquisition module (136, 138, 140, 142, 144) associated with each of the medical sensing modalities of the medical sensing devices and operable to receive patient data associated with each of the medical sensing modalities from the corresponding medical sensing devices, each of the data acquisition modules being further operable to create a plurality of messages containing patient data;
a message queue module (146) operable to receive and temporarily store the plurality of messages from the data acquisition modules; a processing module (150) associated with each of the medical sensing modalities and operable to retrieve the plurality of messages created by the corresponding data acquisition module from the message queue module, to process the patient data within the plurality of messages, and to transmit a second plurality of messages containing the processed patient data to the message queue module;
a user interface module (154) associated with each of the medical sensing modalities and operable to retrieve the second plurality of messages from the message queue module and to present the processed patient data within a graphical user interface; and
a processing stack (148) operable to expose an application programming interface to the processing module.

2. The medical sensing system of claim 1, wherein the processing stack is configured so that the plurality of processing modules are independent of each other in that additional processing modules may be added to the plurality of processing modules without change to existing processing modules in the plurality of processing modules or the processing stack.

3. The medical sensing system of claim 1, wherein the plurality of user interface modules are independent of each other in that additional user interface modules may be added to the plurality of user interface modules without change to existing user interface modules in the plurality of user interface modules or the processing stack.

4. The medical sensing system of claim 1, wherein each of the plurality of messages contains a portion of the patient data and a header.

5. The medical sensing system of claim 4, wherein the information includes information identifying the medical sensing modality.

6. The medical sensing system of claim 1, wherein the processing modules are operable to process the respective patient data concurrently.

7. The medical sensing system of claim 1, wherein the plurality of messages containing the patient data comprise packetized patient data.

8. The medical sensing system of claim 1, wherein the data acquisition modules, the processing modules and user interface modules are each in communication with the message queue module via application programming interfaces.

9. The medical sensing system of claim 1, wherein the medical sensing modalities are one of intravascular ultrasound (IVUS) imaging, intravascular photoacoustic (IVPA) imaging, optical coherence tomography (OCT), forward looking IVUS (FLIVUS), fractional flow reserve (FFR), coronary flow reserve (CFR), and angiography.

10. The medical sensing system of claim 4, wherein the data acquisition modules are operable to associate the information with the portion of the patient data.

11. The medical sensing system of claim 1, the processing system including a user interface stack (152) operable to expose an application programming interface to the user interface modules.

12. The medical sensing system of claim 1, wherein the message queue module is configured to allocate a portion of the message queue to the plurality of messages associated with each of the medical sensing modalities.

13. The medical sensing system of claim 1, wherein the message queue module is configured to make the plurality of messages available based on at least one of:
a First-In-First-Out (FIFO) manner; and
priority values stored in headers of the plurality of messages.

## Patentansprüche

1. Medizinisches Erfassungssystem (100), umfassend ein Verarbeitungssystem (101), das kommunikativ mit einer Vielzahl von ersten medizinischen Erfassungsvorrichtungen (108, 110, 116) gekoppelt ist, wobei das Verarbeitungssystem aufweist:
ein erstes Datenerfassungsmodul (136, 138, 140, 142, 144), das jeder der medizinischen Erfassungsmodalitäten der medizinischen Erfassungsvorrichtungen zugeordnet ist und betrieben werden kann, um erste Patientendaten zu empfangen, die jeder der medizinischen Erfassungsmodalitäten von den entsprechenden medizinischen Erfassungsvorrichtungen zugeordnet sind, wobei jedes der Datenerfassungsmodule ferner betrieben werden kann, um eine erste Vielzahl von Nachrichten zu erzeugen, die Patientendaten enthält;
ein Nachrichtenwarteschlangenmodul (146), das betrieben werden kann, um die erste Vielzahl von Nachrichten von den ersten Datenerfassungsmodulen zu empfangen und vorübergehend zu speichern;
ein erstes Verarbeitungsmodul (150), das jeder der medizinischen Erfassungsmodalitäten zugeordnet ist und betrieben werden kann, um die erste Vielzahl von Nachrichten, die durch das entsprechende Datenerfassungsmodul aus dem Nachrichtenwarteschlangenmodul erzeugt wurde, abzurufen, um die ersten Patientendaten innerhalb der ersten Vielzahl von Nachrichten zu verarbeiten, und um eine zweite Vielzahl von Nachrichten, welche die verarbeiteten ersten Patientendaten enthält, an das Nachrichtenwarteschlangenmodul zu übertragen;
ein erstes Benutzerschnittstellenmodul (154), das jeder der medizinischen Erfassungsmodalitäten zugeordnet ist und betrieben werden kann, um die zweite Vielzahl von Nachrichten von dem Nachrichtenwarteschlangenmodul abzurufen und die verarbeiteten ersten Patientendaten innerhalb einer ersten grafischen Benutzerschnittstelle darzustellen; und
einen Verarbeitungsstapel (148), der betrieben werden kann, um eine Anwendungsprogrammierschnittstelle für das erste Verarbeitungsmodul verfügbar zu machen.

2. Medizinisches Erfassungssystem nach Anspruch 1, wobei der Verarbeitungsstapel konfiguriert ist, damit die Vielzahl von Verarbeitungsmodulen unabhängig voneinander ist, indem zusätzliche Verarbeitungsmodule zu der Vielzahl von Verarbeitungsmodulen hinzugefügt werden können,
ohne bestehende Verarbeitungsmodule in der Vielzahl von Verarbeitungsmodulen oder dem Verarbeitungsstapel zu ändern.

3. Medizinisches Erfassungssystem nach Anspruch 1, wobei die Vielzahl von Benutzerschnittstellenmodulen voneinander unabhängig ist, indem zusätzliche Benutzerschnittstellenmodule zu der Vielzahl von Benutzerschnittstellenmodulen hinzugefügt werden können, ohne bestehende Benutzerschnittstellenmodule in der Vielzahl von Benutzerschnittstellenmodulen oder dem Verarbeitungsstapel zu ändern.

4. Medizinisches Erfassungssystem nach Anspruch 1, wobei jede der Vielzahl von Nachrichten einen Teil der ersten Patientendaten und eine Kopfzeile enthält.

5. Medizinisches Erfassungssystem nach Anspruch 4, wobei die Information Information aufweist, welche die erste medizinische Erfassungsmodalität identifiziert.

6. Medizinisches Erfassungssystem nach Anspruch 1, wobei die Verarbeitungsmodule betrieben werden können, um die jeweiligen Patientendaten gleichzeitig zu verarbeiten.

7. Medizinisches Erfassungssystem nach Anspruch 1, wobei die Vielzahl von Nachrichten, welche die Patientendaten enthält, paketierte Patientendaten umfasst.

8. Medizinisches Erfassungssystem nach Anspruch 1, wobei die Datenerfassungsmodule, die Verarbeitungsmodule und die Benutzerschnittstellenmodule jeweils über Anwendungsprogrammierschnittstellen mit dem Nachrichtenwarteschlangenmodul kommunizieren.

9. Medizinisches Erfassungssystem nach Anspruch 1, wobei die medizinischen Erfassungsmodalitäten eine von intravaskulärer Ultraschall (IVUS)-Bildgebung, intravaskulärer photoakustischer (IVPA)-Bildgebung, optischer Kohärenztomographie (OCT), vorwärtsgerichteter IVUS (FLIVUS), Teildurchflussreserve (FFR), koronare Durchflussreserve (CFR) und Angiographie sind.

10. Medizinisches Erfassungssystem nach Anspruch 4, wobei die Datenerfassungsmodule betrieben werden können, um die Information dem Teil der ersten Patientendaten zuzuordnen.

11. Medizinisches Erfassungssystem nach Anspruch 1, wobei das Verarbeitungssystem einen Benutzerschnittstellenstapel (152) aufweist, der betrieben werden kann, um eine Anwendungsprogrammierschnittstelle für die Benutzerschnittstellenmodule verfügbar zu machen.

12. Medizinisches Erfassungssystem nach Anspruch 1, wobei das Nachrichtenwarteschlangenmodul zum Zuweisen eines Teils der Nachrichtenwarteschlange der Vielzahl von Nachrichten konfiguriert ist, die jeder der medizinischen Erfassungsmodalitäten zugeordnet ist.

13. Medizinisches Erfassungssystem nach Anspruch 1, wobei das Nachrichtenwarteschlangenmodul konfiguriert ist, um die erste Vielzahl von Nachrichten basierend auf mindestens einem von Folgendem verfügbar zu machen:
FIFO(First-In-First-Out); und
Prioritätswerte, die in Kopfzeilen der Vielzahl von Nachrichten gespeichert sind.

## Revendications

1. Système de détection médicale (100) comprenant un système de traitement (101) couplé de façon à communiquer avec une pluralité de dispositifs de détection médicale (108, 110, 116), le système de traitement incluant :
un module d'acquisition de données (136, 138, 140, 142, 144) associé à chacune des modalités de détection médicale des dispositifs de détection médicale et pouvant fonctionner de façon à recevoir des données de patient associées à chacune des modalités de détection médicale à partir des dispositifs de détection médicale correspondants, chacun des modules d'acquisition de données pouvant en outre fonctionner de façon à créer une pluralité de messages contenant des données de patient ;
un module de file de messages (146) pouvant fonctionner de façon à recevoir et stocker provisoirement la pluralité de messages des modules d'acquisition de données ;
un module de traitement (150) associé à chacune des modalités de détection médicale et pouvant fonctionner de façon à récupérer la pluralité des messages créés par le module d'acquisition de données correspondant du module de file de messages, pour traiter les données de patient dans la pluralité des messages, et pour transmettre une deuxième pluralité de messages contenant les données de patient traitées au module de file de messages ;
un module d'interface utilisateur (154) associé à chacune des modalités de détection médicale et pouvant fonctionner de façon à récupérer la deuxième pluralité de messages du module de file de messages et à présenter les données de patient traitées dans une interface graphique utilisateur ; et
une pile de traitement (148) pouvant fonctionner de façon à exposer une interface de programmation d'application au module de traitement.

2. Système de détection médicale selon la revendication 1, dans lequel la pile de traitement est configurée de sorte que la pluralité de modules de traitement soient indépendants les uns des autres afin que des modules de traitement supplémentaires puissent être ajoutés à la pluralité de modules de traitement sans modifier des modules de traitement existants dans la pluralité des modules de traitement ou de la pile de traitement.

3. Système de détection médicale selon la revendication 1, dans lequel la pluralité des modules d'interface utilisateur sont indépendants les uns des autres afin que des modules d'interface utilisateur supplémentaires puissent être ajoutés à la pluralité de modules d'interface utilisateur sans modifier des modules d'interface utilisateur existants dans la pluralité des modules d'interface utilisateur ou de la pile de traitement.

4. Système de détection médicale selon la revendication 1, dans lequel la pluralité de messages contient une partie des données du patient et un en-tête.

5. Système de détection médicale selon la revendication 4, dans lequel les informations incluent des informations identifiant la modalité de détection médicale.

6. Système de détection médicale selon la revendication 1, dans lequel les modules de traitement peuvent fonctionner de façon à traiter les données de patient respectives de façon concomitante.

7. Système de détection médicale selon la revendication 1, dans lequel la pluralité de messages contenant les données du patient comprend des données de patient sous forme de paquets.

8. Système de détection médicale selon la revendication 1, dans lequel les modules d'acquisition de données, les modules de traitement et les modules d'interface utilisateur sont chacun en communication avec le module de file de messages via des interfaces de programmation d'application.

9. Système de détection médicale selon la revendication 1, dans lequel les modalités de détection médicale sont l'une parmi une imagerie intravasculaire à ultrasons (IVUS), une imagerie intravasculaire photo-acoustique (IVPA), une tomographie par cohérence optique (OCT), une imagerie intravasculaire à ultrasons à vision frontale (FLIVUS), une FFR (fractional flow reserve), une CRF (coronary flow reserve) et une angiographie.

10. Système de détection médicale selon la revendication 4, dans lequel les modules d'acquisition de données peuvent fonctionner de façon à associer les informations avec la partie de données de patient.

11. Système de détection médicale selon la revendication 1, le système de traitement incluant une pile d'interface utilisateur (152) pouvant fonctionner de façon à exposer une interface de programmation d'application aux modules d'interface utilisateur.

12. Système de détection médicale selon la revendication 1, dans lequel le module de file de messages est configuré de façon à attribuer une partie de la file de messages à la pluralité de messages associés à chacune des modalités de détection médicale.

13. Système de détection médicale selon la revendication 1, dans lequel le module de file de messages est configuré pour produire la pluralité de messages disponibles sur la base d'au moins l'un parmi :
un mode premier entré-premier sorti (PEPS) ; et
des valeurs prioritaires stockées dans des en-têtes de la pluralité des messages.
